# FASCICULE DE BREVET EUROPEEN

(11) **EP 3 556 424 B1**
(45) Date de publication et mention de la délivrance du brevet: **10.04.2024**
(21) Numéro de dépôt: 19169963.6
(22) Date de dépôt: 17.04.2019
(51) Int. Cl.: A61M 37/00, A61B 90/00

(54) **DISPOSITIF D'INJECTION D'IMPLANT DOTÉ DE MOYENS DE POUSSÉE À LIBÉRATION SÉQUENTIELLE**
INJEKTIONSVORRICHTUNG EINES IMPLANTATS, DAS MIT MITTELN ZUM DRÜCKEN ZUR SEQUENTIELLEN FREISETZUNG AUSGESTATTET IST
IMPLANT INJECTION DEVICE PROVIDED WITH SEQUENTIAL THRUST RELEASE MEANS

(30) Priorité: 18.04.2018 FR 1853405
(43) Date de publication de la demande: 23.10.2019
(73) Titulaire: NEMERA LA VERPILLIERE, 38290 La Verpillière (FR)
(72) Inventeur: PAINCHAUD, Gaëtan, 69340 FRANCHEVILLE (FR); DUGAND, Pascal, 38780 ESTRABLIN (FR); MEGARD, Thomas, 71960 LA ROCHE-VINEUSE (FR)
(74) Mandataire: LLR

(56) Documents cités:
- US-A- 4 994 028
- US-A1- 2004 127 765
- US-A1- 2016 296 739
- US-B1- 6 270 472

## Description

L'invention concerne le domaine technique de l'injection d'un ou plusieurs implants dans le corps d'un patient.

On connaît les documents suivants : le document US4994028A divulgue un dispositif d'implantation sous-cutanée d'implants ; le document US6270472B1 divulgue un appareil et un procédé pour déposer une pluralité d'implants dans des tissus mous ; le document US20040127765A1 divulgue des appareils et procédés d'implantation percutanée d'objets, comme des marqueurs ou sources radioactives ; le document US20160296739A1 divulgue un dispositif de distribution d'implants utilisé pour insérer une pluralité d'implants dans une cavité ou une incision. On connaît en particulier du document US20090281520A1 un dispositif d'injection d'implant dans lequel l'injection de l'implant peut se faire par appui coulissant sur un bouton, le bouton étant alors entraîné par l'utilisateur dans une direction sensiblement parallèle à la direction d'injection. Ainsi, avant actionnement, la tige de poussée est rétractée et le bouton se trouve du côté de l'extrémité proximale du dispositif, c'est-à-dire l'extrémité opposée à l'extrémité d'injection. Lors de l'appui coulissant sur le bouton par l'utilisateur, le bouton coulisse dans une direction sensiblement parallèle à la direction d'injection et entraîne la tige de poussée, laquelle pousse l'implant et permet son injection.

Toutefois, un tel actionnement par appui coulissant ne permet pas aisément à l'utilisateur d'injecter plusieurs implants. En effet, cet actionnement est généralement réalisé par le pouce de l'utilisateur, lequel possède une course réduite pour l'actionnement. Cette course réduite n'est pas suffisante pour permettre l'injection successive de deux implants. De plus un tel actionnement ne permet pas de maintenir une grande précision d'injection sur une grande course, et ne permet pas en particulier de stopper l'injection lorsque l'injection d'un implant est effectuée, par exemple pour orienter l'aiguille d'injection dans une autre direction pour un second implant afin de ne pas injecter sur une trop longue profondeur. En effet, le risque de débuter de manière non souhaitée l'injection d'un implant suivant l'implant déjà injecté existe avec un tel actionnement. Cela n'est pas souhaitable pour garantir une injection correcte, notamment pour éviter une rupture d'au moins un des implants ou une blessure du patient.

Ainsi, compte tenu de leur type d'actionnement, ces dispositifs d'injection d'implant ne permettent pas d'injecter aisément une pluralité d'implants.

La présente invention vise notamment à fournir un dispositif d'injection d'implant, lequel permet d'injecter aisément une pluralité d'implants tout en maîtrisant l'injection de chaque implant. L'invention est définie dans les revendications 1 à 16. A cet effet, l'invention a pour objet un dispositif d'injection d'implant, comportant :
- une aiguille d'injection,
- un corps de réception d'au moins un premier implant et un second implant,
- des moyens d'injection, comprenant :
   . une tige de poussée, disposée en amont desdits implants logés dans le corps de réception et configurée pour pousser le premier implant et le second implant au travers de l'aiguille d'injection entre une position initiale et une position finale dans laquelle le premier implant et le second implant sont injectés,
   . des moyens de poussée sur la tige de poussée, configurés pour exercer une force tendant à faire passer la tige de poussée de la position initiale à la position finale,
   . des moyens de butée intermédiaire permettant de maintenir la tige de poussée dans une position intermédiaire entre la position initiale et la position finale, lesquels s'opposent à la force exercée par les moyens de poussée lorsque la course de la tige de poussée atteint une distance prédéterminée correspondant à la longueur d'injection du premier implant,
   . des moyens d'actionnement par un utilisateur, configurés pour libérer un déplacement de la tige de poussée de la position intermédiaire vers la position finale.

Ainsi, on propose de réaliser l'injection séquentiellement, en considérant l'injection de chaque implant comme une séquence débutant par un déplacement de la tige de poussée, les moyens de poussée agissant alors sur la tige de poussée pour permettre l'injection d'un premier implant, et se terminant par l'arrêt des moyens de poussée par les moyens de butée de manière à éviter l'injection de l'implant suivant, appelé deuxième implant. En effet, pour chaque injection respective d'implant, l'utilisateur actionne les moyens d'actionnement, lesquels libèrent un déplacement de la tige de poussée, ce qui permet l'injection de l'implant respectif. Lorsque la course de la tige de poussée correspond à la longueur d'injection respective souhaitée pour cet implant respectif, les moyens de poussée sont arrêtés par les moyens de butée, sans nécessiter pour l'utilisateur de contrôler ou d'actionner un quelconque élément. L'attention de l'utilisateur peut ainsi être focalisée sur le positionnement de l'aiguille d'injection et ainsi sur le positionnement de l'implant respectif dans le corps du patient. L'utilisateur commande facilement et à la demande la libération de chaque implant via les moyens d'actionnement.

On comprend qu'il est particulièrement intéressant de pouvoir injecter des implants de façon séquentielle. En particulier, cela peut permettre à l'utilisateur activant le bouton d'actionnement de reprendre sa course et donc de ne pas perdre en précision lors de l'injection de l'implant suivant. Ou encore cela permet de modifier l'orientation de l'aiguille afin d'injecter un deuxième implant dans une direction autre que celle du premier implant et ainsi éviter une injection sur une trop longue profondeur, ce qui pourrait être le cas lors d'une injection continue des implants.

On entend de préférence par "implant", un composé pharmaceutique à l'état solide ou semi-solide, par exemple sous forme de liquide encapsulé, et/ou un composant électronique, par exemple une puce électronique pouvant être de type RFID. On entend généralement par "patient" ou "sujet" un être vivant comme par exemple un mammifère, notamment un être humain. L'utilisateur est en général une personne différente du patient, mais il est possible que l'utilisateur soit le patient lui-même.

Dans la présente description, on comprend que la direction distale désigne la direction la plus éloignée des doigts d'un utilisateur, c'est-à-dire la plus proche de la peau ou de la surface d'un patient au moment d'une injection, et la direction proximale désigne la direction opposée à la direction distale. En d'autres termes, on considère que la direction distale et le sens distal sont la direction et le sens qui vont vers «l'avant » du dispositif d'injection d'implant, direction autrement appelée direction d'injection. En particulier, l'extrémité distale d'une pièce correspond à l'extrémité se trouvant du côté de l'aiguille d'injection, et l'extrémité proximale correspond à l'extrémité opposée. On comprend par ailleurs que l'axe d'injection, portant la direction d'injection, correspond à l'axe du dispositif d'injection d'implant, défini par l'axe de l'aiguille d'injection.

On comprend par conséquent que la direction « aval » est une direction opposée à la direction « amont » et correspond à la direction orientée dans le sens de l'extrémité distale du dispositif d'injection d'implant, c'est-à-dire en direction du site d'injection, vers l'extrémité configurée pour se trouver en contact avec le site d'injection de l'implant. Ainsi, la direction « aval » peut également être appelée direction d'injection. On comprend que les termes « amont » et « aval » sont relatifs aux directions distale et proximale, un élément aval étant disposé plus loin dans la direction distale qu'un élément amont.

Avantageusement, les moyens de butée sont configurés de façon à générer une indication sensorielle, telle qu'une indication tactile ou sonore, lorsque la tige de poussée atteint sa position intermédiaire de maintien par les moyens de butée intermédiaire.

Le dispositif d'injection d'implant peut en outre comporter l'une ou plusieurs des caractéristiques suivantes, prises seules ou en combinaison.
- Le dispositif d'injection d'implant comprend des moyens de butée initiale s'opposant à la force exercée par les moyens de poussée et permettant de maintenir la tige de poussée dans la position initiale.

Ainsi, grâce aux moyens de butée initiale, l'injection d'un premier implant ne peut être déclenchée par accident.
- Les moyens d'actionnement comportent un bouton d'actionnement, lequel est mobile par appui par un utilisateur entre :
   . une position de repos avant actionnement,
   . une position médiane, dans laquelle les moyens d'actionnement libèrent le déplacement de la tige de poussée depuis sa position initiale vers sa position intermédiaire, et
   . une position terminale, dans laquelle les moyens d'actionnement libèrent le déplacement de la tige de poussée depuis sa position intermédiaire vers sa position finale.

Ainsi, l'actionnement est réalisé séquentiellement et ce, de manière particulièrement simple par l'utilisateur.
- Les moyens d'actionnement comportent un élément d'indication configuré pour indiquer à un utilisateur que le bouton d'actionnement est dans sa position médiane, l'élément d'indication comprenant de préférence un bouton secondaire étant à affleurement du bouton d'actionnement lorsque ce dernier est dans sa position médiane.

Ainsi, grâce à l'élément d'indication, l'utilisateur visualise ou détecte aisément à quel stade de l'injection le dispositif d'injection d'implant se trouve. On peut bien sûr envisager d'autres types d'élément d'indication. Un élément d'indication tactile et/ou sonore est particulièrement avantageux, car cette indication est très pratique pour que l'utilisateur n'ait pas à regarder le dispositif d'injection d'implant et puisse concentrer son regard sur la zone d'injection.
- Les moyens d'actionnement comportent un moyen de rappel du bouton d'actionnement vers sa position de repos, notamment une languette élastique portée par le bouton d'actionnement, ou un ressort.

Ainsi, l'utilisateur doit fournir un certain effort pour actionner le bouton d'actionnement, ce qui évite de provoquer l'injection d'un implant par accident, par exemple pour orienter l'aiguille d'injection dans une autre direction pour un autre implant ultérieur comme un second implant, afin de ne pas injecter sur une trop longue profondeur.
- Le dispositif d'injection d'implant comporte des moyens anti-retour empêchant le retour du bouton d'actionnement respectivement depuis sa position médiane et depuis sa position terminale.

Ainsi, lorsque l'injection d'un implant respectif est achevée, le bouton d'actionnement ne peut être actionné que pour l'injection d'autres implants disposés en amont, ou bien ne peut plus être utilisé si l'implant respectif était le dernier implant à injecter. De cette manière, le bouton d'actionnement est actionné uniquement sur la course nécessaire à l'injection de l'implant qui suit. Par ailleurs, le dispositif d'injection d'implant n'est pas réutilisable, permettant de respecter les contraintes d'hygiènes relatives à un tel dispositif d'injection d'implant.
- Les moyens d'actionnement sont latéraux, par exemple mobiles par coulissement dans une direction radiale par rapport à l'axe longitudinal de la tige de poussée ou par pivotement autour d'un axe orthogonal à l'axe longitudinal de la tige de poussée.

En d'autres termes, l'utilisateur appuie perpendiculairement à la direction d'injection, à la différence d'un appui axial, notamment sur l'extrémité proximale du dispositif d'injection d'implant. Ainsi, l'actionnement du dispositif d'injection d'implant est aisé pour un utilisateur, du fait que l'effort n'est pas à exercer parallèlement à la direction d'injection.
- Les moyens de butée intermédiaire comportent un organe de butée axiale intermédiaire permettant de maintenir la tige de poussée dans la position intermédiaire, l'organe de butée axiale intermédiaire étant configuré pour être entraîné par les moyens d'actionnement pour libérer un déplacement de la tige de poussée de la position intermédiaire vers la position finale.

Ainsi, la sécurité du dispositif d'injection d'implant est augmentée. L'utilisation d'un organe de butée axiale intermédiaire permet de stopper la course de la tige de poussée et par conséquent l'injection, une fois qu'un implant est injecté.
- Les moyens de butée initiale comportent un organe de butée axiale initiale permettant de maintenir la tige de poussée dans la position initiale, l'organe de butée axiale initiale étant configuré pour être entraîné par les moyens d'actionnement pour libérer un déplacement de la tige de poussée de la position initiale vers la position intermédiaire.

Ainsi, la sécurité du dispositif d'injection d'implant est augmentée. L'utilisation d'un organe de butée axiale initiale permet de maintenir la tige de poussée dans sa position initiale et par conséquent d'empêcher l'injection d'un premier implant.
- Les moyens d'actionnement provoquent la rotation de l'un au moins parmi l'organe de butée axiale initiale et l'organe de butée axiale intermédiaire pour libérer respectivement un déplacement de la tige de poussée de la position initiale vers la position intermédiaire et/ou de la position intermédiaire vers la position finale.

Ainsi, on propose d'utiliser une transmission de mouvement de type came, pour que l'actionnement par appui de l'utilisateur se transforme en rotation libérant le déplacement de la tige de poussée, permettant ainsi de limiter la course à fournir par l'utilisateur, par exemple par un doigt de l'utilisateur.
- L'organe de butée axiale intermédiaire et l'organe de butée axiale initiale sont d'un seul tenant formant un organe de butée axiale, et sont formés par un cylindre en partie évidé et orienté sensiblement longitudinalement, dont la paroi latérale comporte une pluralité de décrochages successifs sous forme de marches d'escaliers.

Ainsi, les moyens de butée initiale et les moyens de butée intermédiaires sont réalisés de manière particulièrement simple.
- Le dispositif d'injection d'implant comporte des moyens de butée finale s'opposant à la force exercée par les moyens de poussée et permettant de maintenir la tige de poussée dans la position finale.
- Les moyens de butée finale comportent un organe de butée axiale finale permettant de maintenir la tige de poussée dans la position finale.
- L'organe de butée axiale initiale, l'organe de butée axiale intermédiaire et l'organe de butée axiale finale sont d'un seul tenant formant un organe de butée axiale, et sont formés par un cylindre en partie évidé et orienté sensiblement longitudinalement, dont la paroi latérale comporte une pluralité de décrochages successifs sous forme de marches d'escaliers.
- La tige de poussée vient en contact en fin de course, c'est-à-dire dans sa position finale, contre une butée axiale formée par le fond de l'un des décrochages successifs de la paroi latérale de l'organe de butée axiale.

Ainsi, les moyens de butée initiale, les moyens de butée intermédiaires et les moyens de butée finale sont réalisés de manière particulièrement simple.
- L'un au moins parmi l'organe de butée axiale initiale et l'organe de butée axiale intermédiaire comporte un pion disposé sur son extrémité distale, excentré par rapport à l'axe longitudinal de la tige de poussée, coopérant avec les moyens d'actionnement pour provoquer la rotation de l'un au moins parmi l'organe de butée axiale initiale et l'organe de butée axiale intermédiaire.

La fabrication d'un tel organe de butée axiale est ainsi particulièrement simple. On comprend que, du fait que le pion est excentré, il peut être poussé dans la direction transversale par les moyens d'actionnement, générant ainsi la rotation autour de l'axe longitudinal de la tige de poussée de l'un au moins parmi l'organe de butée axiale initiale et l'organe de butée axiale intermédiaire,
- Le dispositif d'injection d'implant comprend un boîtier de préhension et un support portant la tige de poussée monté coulissant par rapport au boîtier de préhension, de préférence une douille coulissante coopérant avec une rainure portée par le boîtier de préhension, le support comprenant une surface d'appui destinée à coopérer avec les moyens de butée intermédiaire pour maintenir axialement la tige de poussée dans la position intermédiaire.

Ainsi, le fonctionnement d'un tel dispositif d'injection d'implant est simplifié et plus fiable. L'utilisation d'un support de la tige de poussée est particulièrement intéressant en combinaison avec l'utilisation de moyens de poussée tels qu'un ressort, du fait que ce support peut servir de surface d'appui du ressort, plus grande que l'extrémité de la tige de poussée. Par ailleurs, le support monté coulissant dans le boîtier de préhension permet de guider et centrer la tige de poussée, pour éviter qu'elle dévie sous la poussée du ressort.
- Les moyens de poussée comportent un ressort de poussée, prenant appui entre un boîtier de préhension et la tige de poussée, de préférence disposé entre le boîtier de préhension et le support portant la tige de poussée.

Le ressort de poussée peut travailler en traction ou en compression. L'utilisation d'un ressort de poussée pour exercer la poussée sur la tige de poussée est particulièrement intéressante du fait que la force du ressort de poussée permet de faciliter et rendre plus précise l'injection, tout en étant limitée par au moins les moyens de butée intermédiaire, et éventuellement les moyens de butée initiale et/ou finale, afin de ne pas injecter trop lentement ou trop rapidement un implant, tout en laissant à l'utilisateur le contrôle du déclenchement de l'injection d'un implant. C'est tout particulièrement avantageux pour injecter une pluralité d'implants. La force maximale exercée sur la tige de poussée est définie afin d'éviter d'endommager la tige de poussée et/ou un implant.
- Le dispositif d'injection d'implant comporte un élément de blocage amovible des moyens de poussée, configuré pour maintenir le dispositif d'injection d'implant dans une position de stockage, en particulier dans laquelle la tige de poussée occupe sa position initiale.

Ainsi, le transport et la manipulation du dispositif d'injection d'implant préalablement à son utilisation sont plus sûrs, le dispositif d'injection d'implant ne pouvant être actionné par inadvertance ou à cause d'une chute de celui-ci.
- Le dispositif d'injection d'implant comporte des moyens de verrouillage agencés pour bloquer la tige de poussée dans sa position finale, position dans laquelle la tige de poussée fait de préférence saillie vers la direction aval au-delà de l'extrémité de l'aiguille d'injection.

Ainsi, le dispositif d'injection d'implant n'est pas réutilisable, permettant de respecter les contraintes d'hygiènes relatives à un tel dispositif d'injection d'implant.
- Le dispositif d'injection d'implant peut injecter une pluralité d'implants et comprend une pluralité de moyens de butée intermédiaire respectifs permettant de maintenir la tige de poussée dans une pluralité de positions intermédiaires respectives, lesquels s'opposent à la force exercée par les moyens de poussée lorsque la course de la tige de poussée atteint une distance prédéterminée respective correspondant à la longueur d'injection d'un implant respectif.

Ainsi, le dispositif d'injection d'implant peut être adapté à l'injection d'un nombre n quelconque d'implants, par exemple en y intégrant un nombre n-1 de moyens de butée intermédiaire, lesquelles permettent de stopper le déplacement de la tige de poussée après chaque injection d'implant, puis de libérer le déplacement de la tige de poussée de la position intermédiaire respective vers une position intermédiaire ultérieure ou bien vers la position finale. En effet, un tel dispositif d'injection d'implant peut être configuré pour injecter plus de deux implants, par exemple entre 3 et 50 implants, plus précisément entre 3 et 10 implants, encore plus précisément exactement 3, 4 ou 5 implants.
- L'aiguille d'injection est solidaire du boîtier de préhension.

Ainsi, lors de l'utilisation du dispositif d'injection d'implant par un utilisateur, l'aiguille d'injection reste fixe par rapport au boîtier de préhension. En particulier, elle ne se rétracte ni lors de l'injection de chaque implant de la pluralité d'implants, ni après l'injection de chaque implant de la pluralité d'implants. Par conséquent, cela permet de simplifier la conception du dispositif d'injection d'implant.
- Les moyens de poussée sont automatiques.

Ainsi, un utilisateur n'a pas à exercer une force de poussée dans une direction distale sur la tige de poussée, ce qui permet d'améliorer la précision de l'actionnement par l'utilisateur des moyens d'actionnement. Par conséquent, cela entraîne l'amélioration de la précision du processus d'injection.
- La tige de poussée est configurée pour pousser le premier implant et le second implant au travers de l'aiguille d'injection entre une position initiale proximale et une position finale distale dans laquelle le premier implant et le second implant sont injectés.

Ainsi, lors de l'utilisation du dispositif d'injection d'implant par un utilisateur, la tige de poussée n'est déplacée que dans la direction distale entre la position initiale et la position finale. Par conséquent, cela entraîne l'amélioration de la précision du processus d'injection.

L'invention sera mieux comprise à la lecture de la description qui va suivre, donnée uniquement à titre d'exemple et faite en se référant aux dessins dans lesquels :
- la figure 1 est une vue en perspective d'un dispositif d'injection d'implant selon un mode de réalisation, en configuration de stockage avant injection ;
- les figures 2 et 3 sont des vues de côté et en perspective d'une partie du dispositif d'injection d'implant de la figure 1, dans lequel la tige de poussée est dans une position initiale ;
- les figures 4 et 5 sont des vues de côté et en perspective d'une partie du dispositif d'injection d'implant de la figure 1, dans lequel la tige de poussée est dans une position intermédiaire ;
- la figure 6 est une vue de côté et en perspective d'une partie du dispositif d'injection d'implant de la figure 1, dans lequel la tige de poussée est dans une position finale ;
- la figure 7 est une vue de côté et en perspective d'une partie de boîtier de préhension du dispositif d'injection d'implant de la figure 1 ;
- la figure 8 est une vue de côté et en perspective d'une autre partie de boîtier de préhension du dispositif d'injection d'implant de la figure 1.

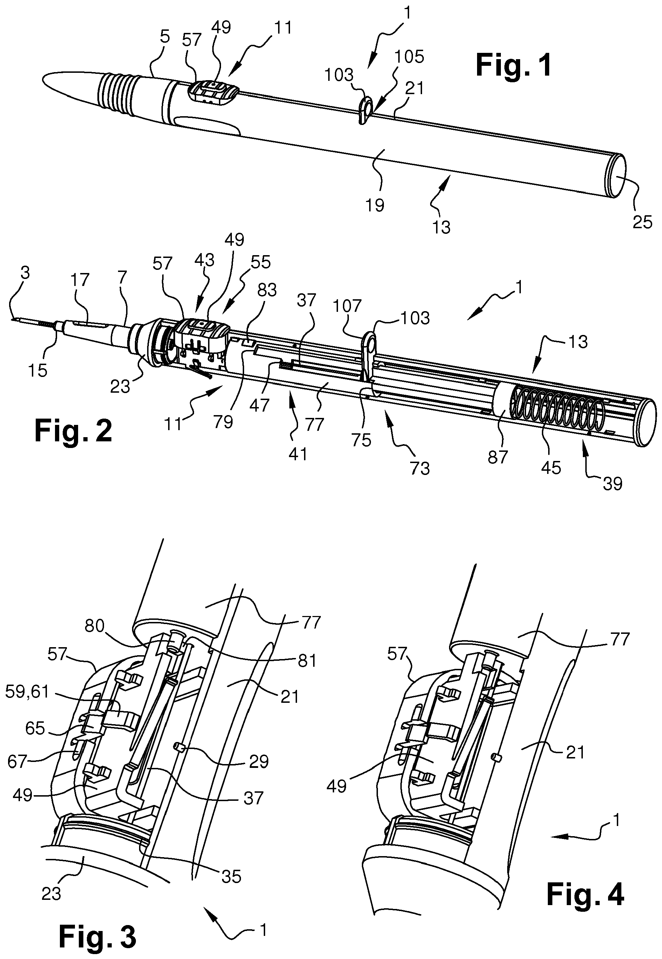

Comme cela est représenté sur les figures 1 et 2, un dispositif d'injection d'implant 1, comporte une aiguille d'injection 3 protégée par un capuchon 5, un corps de réception 7 d'au moins un premier implant et un second implant 9, des moyens d'injection 11 et un boîtier de préhension 13.

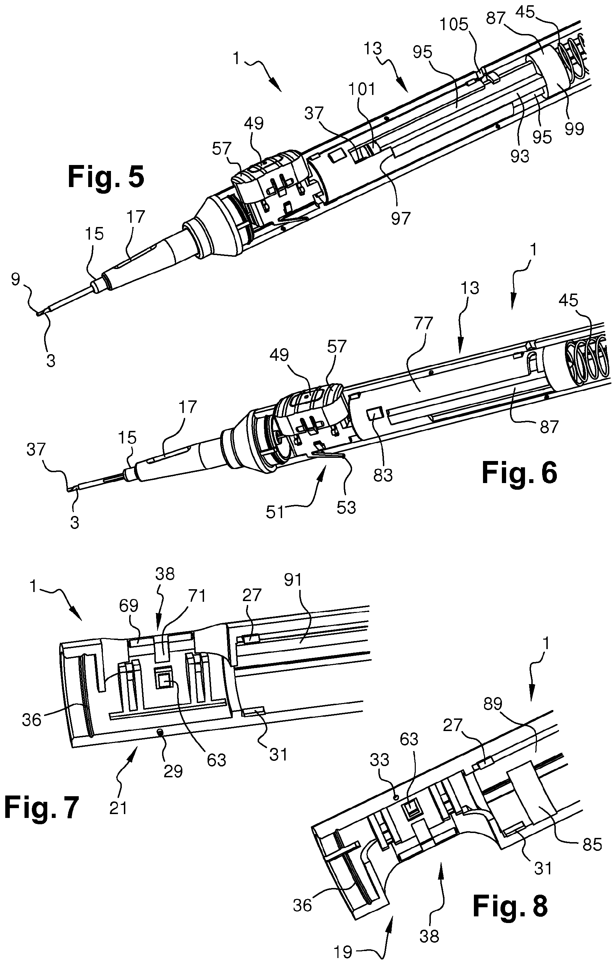

Le dispositif d'injection d'implant 1 est configuré pour injecter plusieurs implants dans le corps d'un patient via l'aiguille d'injection 3 visible sur la figure 2. Dans l'exemple illustré, le dispositif d'injection d'implant 1 est configuré pour injecter un premier implant et un second implant 9 (visible sur la figure 5) dans le corps d'un patient via l'aiguille d'injection 3. Bien que dans ce qui suit, l'exemple soit illustré avec deux implants, le dispositif d'injection d'implant 1 est applicable également à un nombre d'implants supérieur à deux, comme par exemple trois, quatre, cinq, dix implants.

Comme cela est représenté sur les figures 2, 5 et 6, l'aiguille d'injection 3 est creuse et est par exemple réalisée en métal comme de l'acier inoxydable. L'aiguille d'injection 3 comporte une extrémité distale biseautée pour faciliter son insertion dans le corps du patient. L'aiguille d'injection 3 porte un élément support 15, lequel peut être réalisé en matériau plastique et est destiné à limiter la profondeur d'insertion de l'aiguille d'injection 3 dans le corps du patient. L'aiguille d'injection 3 est rapportée à son extrémité proximale sur le corps de réception 7. Elle peut être protégée en configuration de stockage par le capuchon 5.

Le capuchon 5 est un capuchon de protection de l'aiguille d'injection 3, il est ici assemblé sur le boîtier de préhension 13, par encliquetage de son extrémité proximale sur le boîtier de préhension 13. Cependant, d'autres moyens d'assemblage sont possibles, par exemple par vissage. Le capuchon 5 est dans cet exemple en forme d'ogive, pourvue de reliefs de manière à faciliter sa préhension.

Le corps de réception 7 est un corps de réception d'un premier implant et d'un second implant 9. Comme illustré sur la figure 2, le corps de réception 7 est de forme générale tubulaire et/ou tronconique, et loge les implants dans son espace interne, de telle sorte que les implants soient orientés en direction de l'aiguille d'injection 3, face à l'extrémité proximale de l'aiguille d'injection 3. En d'autres termes, le corps de réception 7 est disposé en amont de l'aiguille d'injection 3, et est destiné à recevoir les implants de sorte que les implants soient disposés en amont de l'aiguille d'injection 3, dans la direction d'injection de l'aiguille d'injection 3. Le corps de réception 7 est configuré pour contenir deux implants, les deux implants étant disposés l'un derrière l'autre, c'est-à-dire l'un en amont de l'autre, selon la direction d'injection. Dans l'exemple illustré, le second implant 9 est disposé en amont du premier implant. Le corps de réception 7 peut comporter, à son extrémité distale, un moyen de retenue d'implant, comme une membrane ou un faible rétrécissement de son diamètre interne, ayant pour but d'éviter la chute d'un implant à travers l'aiguille d'injection 3, par exemple sous l'action de la force de gravité terrestre. Le corps de réception 7 peut alternativement comporter, à son extrémité distale, un élément souple de retenue d'implant comportant un orifice d'un diamètre inférieur à celui des implants, lequel est configuré pour se déformer et permettre le passage des implants vers l'aiguille d'injection 3 lors de l'injection. Le corps de réception 7 comporte avantageusement une fenêtre 17, également appelée lumière. Ainsi, un utilisateur est en mesure de détecter visuellement, par la fenêtre 17, la présence des implants dans le dispositif d'injection d'implant 1 avant de réaliser l'injection sur un patient, lorsque le capuchon 5 est ôté. On notera que dans cet exemple, l'aiguille d'injection 3 et le corps de réception 7 sont des pièces rapportées l'une par rapport à l'autre, néanmoins il serait envisageable que l'aiguille d'injection 3 et le corps de réception 7 forment deux parties d'une même pièce, par exemple en étant venus de matière. Par ailleurs, le corps de réception 7 est rapporté à son extrémité proximale sur le boîtier de préhension 13, par exemple par encliquetage de son extrémité proximale sur le boîtier de préhension 13. Ainsi, l'aiguille d'injection est solidaire du boîtier du préhension 13. Le corps de réception 7 comporte ainsi une nervure périphérique, laquelle s'engage dans une rainure correspondante portée par le boîtier de préhension 13. Cependant, d'autres moyens d'assemblage sont possibles, par exemple par vissage. Selon une variante, le corps de réception 7 pourrait être réalisé directement dans le boîtier de préhension 13, en étant venu de matière avec ce dernier. Dans cet exemple, le corps de réception 7 est réalisé en un matériau plastique, éventuellement partiellement ou totalement transparent.

Dans l'exemple illustré, le boîtier de préhension 13 se compose de plusieurs éléments :
- deux demi-coques 19, 21, illustrées notamment sur la figure 1,
- un bouchon aval 23, illustré notamment sur la figure 2, et
- un bouchon amont 25, illustré sur la figure 1.

A l'état assemblé, les deux demi-coques 19, 21, le bouchon aval 23 et le bouchon amont 25 sont maintenus en position les uns par rapport aux autres.

Chaque demi-coque 19, 21 visible sur les figures 7 et 8 comporte des moyens d'assemblage, par exemple des pattes 27 et des pions 29. Chaque demi-coque 19, 21 comporte en outre des logements 31 et trous de réception 33 destinés à recevoir respectivement, à l'état assemblé par encliquetage, les pattes 27 et pions 29 de l'autre demi-boîtier 21, 19.

Par ailleurs à l'état assemblé, le bouchon aval 23 se trouve en butée contre l'extrémité distale des deux demi-coques 19, 21, et est assemblé aux deux demi-coques 19, 21 par encliquetage d'une nervure périphérique 35 comme illustré notamment sur la figure 3, dans une rainure périphérique interne 36 correspondante de chacune des deux demi-coques 19, 21.

De manière similaire, le bouchon amont 25 se trouve en butée contre l'extrémité proximale des deux demi-coques 19, 21, et est assemblé aux deux demi-coques 19, 21 par encliquetage d'une nervure périphérique dans une rainure périphérique interne correspondante de chacune des deux demi-coques 19, 21.

Enfin à l'état assemblé, les deux demi-coques 19, 21 logent les moyens d'injection 11 et comprennent une ouverture 38 par laquelle fait saillie au moins une partie des moyens d'injection 11.

Les moyens d'injection 11 comprennent une tige de poussée 37, des moyens de poussée 39, des moyens de butée intermédiaire 41 et des moyens d'actionnement 43 par un utilisateur.

La tige de poussée 37 est disposée en amont des implants 9 logés dans le corps de réception 7. La tige de poussée 37 s'étend longitudinalement et est configurée pour pousser le premier implant et le second implant 9 au travers de l'aiguille d'injection 3 entre une position initiale et une position finale dans laquelle le premier implant et le second implant 9 sont injectés. Plus précisément dans cet exemple, la tige de poussée 37 est configurée pour pousser le premier implant et le second implant 9 au travers de l'aiguille d'injection 3 entre une position initiale proximale et une position finale distale dans laquelle le premier implant et le second implant 9 sont injectés. Ainsi, dans la position initiale de la tige de poussée 37, le premier implant et le second implant 9 sont logés dans le corps de réception 7, et dans la position finale de la tige de poussée 37, le premier implant et le second implant 9 sont passés à travers l'aiguille d'injection 3 et se trouvent a priori placés dans le corps d'un patient. La tige de poussée 37 peut être réalisée en métal, par exemple en acier, de préférence en acier inoxydable.

Les moyens de poussée 39 sont dans cet exemple automatiques, et comportent dans cet exemple un ressort de poussée 45, lequel prend appui entre le boîtier de préhension 13 et la tige de poussée 37. Les moyens de poussée 39, grâce à l'action du ressort de poussée, 45, sont ainsi configurés pour exercer une force tendant à faire passer la tige de poussée 37 de la position initiale à la position finale. En particulier, comme illustré sur la figure 2, le ressort de poussée 45 est un ressort travaillant en compression. Alternativement, le ressort de poussée peut être un ressort travaillant en traction.

Les moyens de butée intermédiaire 41 permettent de maintenir la tige de poussée 37 dans une position intermédiaire entre la position initiale et la position finale. Les moyens de butée intermédiaires 41 s'opposent à la force exercée par les moyens de poussée 39 lorsque la course de la tige de poussée 37 atteint une distance prédéterminée correspondant à la longueur d'injection du premier implant.

Les moyens de butée intermédiaire 41 comportent un organe de butée axiale intermédiaire 47 permettant de maintenir la tige de poussée 37 dans la position intermédiaire, l'organe de butée axiale intermédiaire 47 étant configuré pour être entraîné par les moyens d'actionnement 43 pour libérer un déplacement de la tige de poussée 37 de la position intermédiaire vers la position finale.

Les moyens d'actionnement 43 par un utilisateur, configurés pour actionner un déplacement de la tige de poussée 37 de la position initiale vers la position finale, comportent un bouton d'actionnement 49, lequel est mobile par appui par un utilisateur entre :
- une position de repos avant actionnement,
- une position médiane, dans laquelle les moyens d'actionnement 43 libèrent le déplacement de la tige de poussée 37 depuis sa position initiale vers sa position intermédiaire, et
- une position terminale, dans laquelle les moyens d'actionnement 43 libèrent le déplacement de la tige de poussée 37 depuis sa position intermédiaire vers sa position finale.

Le bouton d'actionnement 49 est placé dans l'ouverture 38 ménagée dans les deux demi-coques 19, 21 de manière à permettre son actionnement par un utilisateur. De cette manière, les moyens d'actionnement 43, en particulier le bouton d'actionnement 49, sont latéraux, mobiles par coulissement dans une direction radiale par rapport à l'axe longitudinal de la tige de poussée 37. Alternativement, les moyens d'actionnement 43 peuvent être mobiles par pivotement autour d'un axe orthogonal à l'axe longitudinal de la tige de poussée 37 ou bien par pivotement autour de l'axe longitudinal de la tige de poussée 37, et comportent par exemple une bague pivotante.

Les moyens d'actionnement 43 comportent un moyen de rappel 51 du bouton d'actionnement 49 vers sa position de repos, visible sur la figure 6, notamment une languette élastique 53 portée par le bouton d'actionnement 49. En particulier dans cet exemple, le bouton d'actionnement 49 comporte deux languettes élastiques 53 s'étendant depuis une surface du bouton d'actionnement, opposée à une surface d'appui par un utilisateur, et en regard d'une paroi interne des demi-coques 19, 21. Parmi ces deux languettes élastiques 53, l'une s'étend en direction aval depuis une partie amont de cette surface, et l'autre s'étend en direction amont depuis une partie aval de cette surface. Alternativement, le moyen de rappel 51 est un ressort.

Les moyens d'actionnement 43 comportent en outre un élément d'indication 55, visible sur la figure 2, configuré pour indiquer à un utilisateur que le bouton d'actionnement 49 est dans sa position médiane. Ainsi, l'élément d'indication 55 comprend un bouton secondaire 57 (visible sur la figure 4) étant à affleurement du bouton d'actionnement 49 lorsque ce dernier est dans sa position médiane.

Le bouton secondaire 57 est disposé autour du bouton d'actionnement 49. Il est monté coulissant par rapport au boîtier de préhension 13 et au bouton d'actionnement 49. Le bouton secondaire 57 est mobile entre deux positions, l'une dans laquelle le bouton secondaire 57 est à affleurement du bouton d'actionnement 49 lorsque ce dernier est dans sa position médiane, et l'autre dans laquelle le bouton secondaire 57 est à affleurement du bouton d'actionnement 49 lorsque ce dernier est dans sa position terminale. Par « à affleurement », il faut de préférence comprendre que la surface d'appui par un utilisateur sur le bouton secondaire 57 affleure la surface d'appui par un utilisateur sur le bouton d'actionnement 49. En outre, le bouton d'actionnement 49 fait saillie dans sa position de repos par rapport au bouton secondaire 57.

Le dispositif d'injection d'implant 1 comporte également des moyens anti-retour 59 (visibles sur la figure 3) empêchant le retour du bouton d'actionnement 49 respectivement depuis sa position médiane et depuis sa position terminale. Les moyens anti-retour 59 sont en particulier formés par des moyens d'encliquetage. Pour empêcher le retour depuis sa position terminale, les moyens d'encliquetage sont formés par des pattes 61 (visibles sur la figure 3) situées de part et d'autre du bouton d'actionnement 49, lesquelles viennent s'encliqueter dans des logements 63 (visibles sur les figures 7 et 8) situés sur le boîtier de préhension 13, en particulier sur une paroi interne de chaque demi-coque 19, 21. Pour empêcher le retour depuis sa position médiane, les moyens d'encliquetage sont par exemple formés par des godrons situés sur les extrémités amont et aval du bouton d'actionnement 49, lesquels viennent s'encliqueter dans des rainures correspondantes ménagées sur le bouton secondaire 57. Le bouton secondaire 57 est lui-même maintenu en position par rapport au boîtier de préhension 13 par des moyens d'encliquetage comme des pattes 65 (visibles sur la figure 3) et/ou des godrons 67 (visibles sur la figure 3), lesquels viennent s'encliqueter respectivement dans des logements 69 et/ou rainures 71 (visibles sur la figure 7) ménagés sur le boîtier de préhension 13, en particulier sur les demi-coques 19, 21.

Le dispositif d'injection d'implant 1 comporte en outre des moyens de butée initiale 73 (visibles sur la figure 1) s'opposant à la force exercée par les moyens de poussée 39 et permettant de maintenir la tige de poussée 37 dans la position initiale.

Les moyens de butée initiale 73 comportent un organe de butée axiale initiale 75 permettant de maintenir la tige de poussée 37 dans la position initiale, l'organe de butée axiale initiale 75 étant configuré pour être entraîné par les moyens d'actionnement 43 pour libérer un déplacement de la tige de poussée 37 de la position initiale vers la position intermédiaire. En particulier, l'organe de butée axiale initiale 75 et l'organe de butée axiale intermédiaire 47 sont d'un seul tenant et forment un organe de butée axiale 77 comme illustré notamment sur la figure 2, et sont par exemple venus de matière.

L'organe de butée axiale 77 est formé par un cylindre en partie évidé et orienté sensiblement longitudinalement, dont la paroi latérale comporte une pluralité de décrochages successifs 75, 47, 79 sous forme de marches d'escaliers. La tige de poussée 37 vient en contact en fin de course, c'est-à-dire dans sa position finale, contre un organe de butée axiale finale 79 formé par le fond de l'un des décrochages successifs 75, 47, 79 de la paroi latérale de l'organe de butée axiale 77.

Les moyens d'actionnement 43 provoquent la rotation de l'un au moins parmi l'organe de butée axiale initiale 75 et l'organe de butée axiale intermédiaire 47, en particulier de l'organe de butée axiale 77, pour libérer respectivement un déplacement de la tige de poussée 37 de la position initiale vers la position intermédiaire et/ou de la position intermédiaire vers la position finale.

Pour cela, l'un au moins parmi l'organe de butée axiale initiale 75 et l'organe de butée axiale intermédiaire 47, en particulier l'organe de butée axiale 77, comporte un pion 80 (visible sur la figure 3) disposé sur leur extrémité distale, excentré par rapport à l'axe longitudinal de la tige de poussée 37, coopérant avec les moyens d'actionnement 43 pour provoquer la rotation de l'un au moins parmi l'organe de butée axiale initiale 75 et l'organe de butée axiale intermédiaire 47. En particulier, le pion 80 coopère avec une surface 81 sensiblement plane (visible sur la figure 3) du bouton d'actionnement 49.

L'organe de butée axiale 77 comporte une excroissance de butée 83 (visible sur la figure 2), laquelle coopère avec une rainure 85 ménagée de manière périphérique dans la demi-coque 19 comme illustré sur la figure 8, de manière à venir en butée contre l'autre demi-coque 21 lorsque la tige de poussée 37 se trouve dans sa position initiale, comme illustré sur la figure 2. Cela empêche une rotation non voulue de l'organe de butée axiale 77, laquelle pourrait influer sur la libération du déplacement de la tige de poussée 37 depuis la position initiale vers la position intermédiaire.

Le dispositif d'injection d'implant 1 comporte également un support 87 portant la tige de poussée 37. Le support 87 est monté coulissant par rapport au boîtier de préhension 13. En particulier, le support 87 est une douille coulissante coopérant avec une rainure portée par le boîtier de préhension 13.

Dans l'exemple illustré notamment sur la figure 5, le support 87 coopère avec une rainure 89 (visible sur la figure 8) portée par la demi-coque 19 et avec une rainure 91 portée par l'autre demi-coque 21. Le support 87 comporte ainsi une section en forme générale de croix, et présente quatre nervures. Deux nervures 93 opposées coopèrent chacune avec l'une des rainures 89, 91, et deux autres nervures 95 coopèrent par coulissement dans la paroi interne de l'organe de butée axiale 77 pour permettre le coulissement relatif entre le support 87 et l'organe de butée axiale 77.

L'extrémité aval de chaque nervure 93 forme une surface d'appui 97 coopérant avec les moyens de butée intermédiaire 41 pour maintenir axialement la tige de poussée 37 dans la position intermédiaire, comme on peut le voir sur la figure 5. En particulier, la surface d'appui 97 coopère avec l'organe de butée axiale initiale 75 lorsque la tige de poussée 37 est dans la position initiale, avec l'organe de butée intermédiaire 47 lorsque la tige de poussée 37 est dans la position intermédiaire, et avec l'organe de butée axiale finale 79 lorsque la tige de poussée 37 est dans la position finale.

Le support 87 comporte, à son extrémité amont, une bague de réception 99 du ressort de poussée 45, et à son extrémité aval, une bague de support 101 de la tige de poussée 37. Ainsi, le ressort de poussée 45 est disposé entre le boîtier de préhension 13 et le support 87 portant la tige de poussée 37.

Le dispositif d'injection d'implant 1, comme illustré sur la figure 1, comporte en outre un élément de blocage amovible 103 des moyens de poussée 39, configuré pour maintenir le dispositif d'injection d'implant 1 dans une position de stockage, dans laquelle la tige de poussée 37 occupe sa position initiale.

Ainsi, pour maintenir le dispositif d'injection d'implant 1 dans sa position de stockage, l'élément de blocage amovible 103 est engagé à travers une ouverture 105 ménagée dans le boîtier de préhension 13, en particulier de manière complémentaire sur chaque demi-coque 19, 21, et ménagée dans l'organe de butée axiale 77. Comme illustré sur la figure 2, l'élément de blocage amovible 103 est formé à une de ses extrémités par une extrémité de préhension 107. L'autre extrémité est par exemple formée par deux branches en particulier en forme de C, lesquelles sont configurées pour s'engager dans une rainure périphérique ménagée sur le support 87 de la tige de poussée 37. De cette manière, un déplacement du support 87 dans la direction d'injection est empêché, les efforts étant transmis depuis les moyens de poussée 39 formés par le ressort de poussée 45 au support 87, puis du support 87 à l'élément de blocage amovible 103, et de l'élément de blocage amovible 103 au boîtier de préhension 13 au niveau des parois de l'ouverture 105. Ainsi, l'élément de blocage amovible 103 doit être retiré avant d'utiliser le dispositif d'injection d'implant 1 pour injecter les implants dans le corps d'un patient.

Le dispositif d'injection d'implant 1 comporte des moyens de verrouillage agencés pour bloquer la tige de poussée 37 dans sa position finale, position dans laquelle la tige de poussée 37 fait de préférence saillie vers la direction aval au-delà de l'extrémité de l'aiguille d'injection 3, comme illustré sur la figure 6. Ainsi dans cette position finale, un ergot supporté par l'organe de butée axiale 77 coopère avec un évidement porté par le boîtier de préhension 13, empêchant le retour de la tige de poussée 37 dans une direction opposée à la direction d'injection. Cela évite de manière simple la réutilisation du dispositif d'injection d'implant 1 et permet en outre d'éviter une blessure due à l'aiguille d'injection 3, par exemple en cas de chute du dispositif d'injection d'implant 1 après utilisation, grâce au fait que la tige de poussée 37 fait saillie au-delà de l'extrémité de l'aiguille d'injection 3 et est bloquée par ces moyens de verrouillage.

Les éléments du dispositif d'injection d'implant 1 dont le matériau n'est pas précisé dans la présente description peuvent être réalisés en matériau thermoplastique, par exemple en polyéthylène ou en polypropylène.

Un exemple de fonctionnement du dispositif d'injection d'implant 1 va à présent être décrit. Cet exemple ne fait pas partie de l'invention revendiquée et est à titre illustratif.

Le dispositif d'injection d'implant 1 comme illustré sur la figure 1 se trouve en configuration de stockage, avant utilisation.

L'utilisateur doit retirer le capuchon 5 de protection de l'aiguille d'injection 3, comme illustré sur la figure 2 - le dispositif d'injection d'implant 1 étant considéré assemblé - il vérifie la présence des implants par visualisation à travers la fenêtre 17 du corps de réception 7. L'utilisateur doit ensuite retirer l'élément de blocage amovible 103 afin que le déplacement de la tige de poussée 37 de sa position initiale à sa position finale ne soit pas bloqué. Dans cette configuration en position initiale, la nervure 93 du support 87 de la tige de support 37 se trouve en butée contre l'organe de butée axiale initiale 75, et le bouton d'actionnement 49 se trouve en position de repos.

L'aiguille d'injection 3 est ensuite enfoncée dans le corps du patient, et l'utilisateur appuie sur le bouton d'actionnement 49. Le bouton d'actionnement 49 coulisse alors radialement relativement au boîtier de préhension 13, et passe de sa position de repos à sa position médiane. Le pion 80 de l'organe de butée axiale 77 est alors entraîné par le déplacement de la surface 81 du bouton d'actionnement 49. Par le biais du pion 80, l'organe de butée axiale 77 est alors entraîné en rotation, ce qui libère le déplacement du support 87 et de la tige de poussée 37, la tige de poussée 37 passant alors de sa position initiale à sa position intermédiaire. Dans cette configuration en position intermédiaire, la nervure 93 du support 87 de la tige de poussée 37 vient en butée contre l'organe de butée axiale intermédiaire 47, ce qui peut générer une indication tactile ou sonore comme une vibration et/ou un 'clic' sonore, dus au choc de la nervure 93 contre l'organe de butée axiale intermédiaire 47. Le bouton d'actionnement 49 se trouve alors dans sa position médiane dans laquelle le bouton secondaire 57 est à affleurement du bouton d'actionnement 49. Dans cette configuration intermédiaire, un premier implant se trouve à l'état injecté dans le corps du patient. De façon optionnelle, cette étape peut être suivie d'une étape de changement du lieu d'injection ou encore de modification de l'orientation de l'aiguille d'injection 3 afin par exemple que le second implant 9 soit injecté à la même profondeur que le premier implant.

L'utilisateur appuie ensuite sur le bouton d'actionnement 49 et le bouton secondaire 57, les deux étant affleurants. Le bouton d'actionnement 49 et le bouton secondaire 57 coulissent alors radialement relativement au boîtier de préhension 13, et le bouton d'actionnement 49 passe de sa position de médiane à sa position terminale. Le pion 80 de l'organe de butée axiale 77 est alors entraîné par le déplacement de la surface 81 du bouton d'actionnement 49. Par le biais du pion 80, l'organe de butée axiale 77 est alors entraîné en rotation, ce qui libère le déplacement du support 87 et de la tige de support 37, la tige de poussée 37 passant alors de sa position intermédiaire à sa position finale. Dans cette configuration en position intermédiaire, la nervure 93 du support 87 de la tige de poussée 37 vient en butée contre l'organe de butée axiale finale 79, ce qui peut générer une indication tactile ou sonore comme une vibration et/ou un 'clic' sonore, du au choc de la nervure 93 contre l'organe de butée axiale finale 79. Le bouton d'actionnement 49 se trouve alors dans sa position terminale dans laquelle le bouton secondaire 57 est encore à affleurement du bouton d'actionnement 49. Dans cette configuration terminale, un dernier implant, ici le second implant 9, se trouve à l'état injecté dans le corps du patient, et la tige de poussée 37 fait saillie vers la direction aval au-delà de l'extrémité de l'aiguille d'injection 3. L'utilisateur retire enfin le dispositif d'injection d'implant 1 hors du corps du patient.

L'invention n'est pas limitée aux modes de réalisation présentés et d'autres modes de réalisation apparaîtront clairement à l'homme du métier. Bien que l'invention ait été illustrée avec un dispositif d'injection d'implant 1 configuré pour injecter deux implants, l'homme du métier comprendra aisément qu'un tel dispositif d'injection d'implant 1 peut être configuré pour injecter un nombre d'implants supérieur à deux, par exemple, trois, quatre, cinq voire dix implants. Dans ce cas, le nombre de moyens de butée intermédiaire, en particulier d'organes de butée axiale intermédiaire, est choisi en fonction du nombre d'implants à injecter et est égal à ce nombre moins un. Ainsi, l'invention concerne également un dispositif d'injection d'implant du type précité, pouvant injecter une pluralité d'implants et comprenant une pluralité de moyens de butée intermédiaires respectifs permettant de maintenir la tige de poussée dans une pluralité de positions intermédiaires respectives, lesquels s'opposent à la force exercée par les moyens de poussée lorsque la course de la tige de poussée atteint une distance prédéterminée respective correspondant à la longueur d'injection d'un implant respectif.

## Revendications

1. Dispositif d'injection d'implant (1), **caractérisé en ce qu'**il comporte :
- une aiguille d'injection (3),
- un corps de réception (7) d'au moins un premier implant et un second implant (9),
- des moyens d'injection (11), comprenant :
. une tige de poussée (37), disposée en amont desdits implants (9) logés dans le corps de réception (7) et configurée pour pousser le premier implant et le second implant (9) au travers de l'aiguille d'injection (3) entre une position initiale et une position finale dans laquelle le premier implant et le second implant (9) sont injectés,
. des moyens de poussée (39) sur la tige de poussée (37), configurés pour exercer une force tendant à faire passer la tige de poussée (37) de la position initiale à la position finale,
. des moyens de butée intermédiaire (41) permettant de maintenir la tige de poussée (37) dans une position intermédiaire entre la position initiale et la position finale, lesquels s'opposent à la force exercée par les moyens de poussée (39) lorsque la course de la tige de poussée (37) atteint une distance prédéterminée correspondant à la longueur d'injection du premier implant (9),
. des moyens d'actionnement (43) par un utilisateur, configurés pour libérer un déplacement de la tige de poussée (37) de la position intermédiaire vers la position finale.

2. Dispositif d'injection d'implant (1) selon la revendication précédente, comprenant des moyens de butée initiale (73) s'opposant à la force exercée par les moyens de poussée (39) et permettant de maintenir la tige de poussée (37) dans la position initiale.

3. Dispositif d'injection d'implant (1) selon l'une quelconque des revendications précédentes, dans lequel les moyens d'actionnement (43) comportent un bouton d'actionnement (49), lequel est mobile par appui par un utilisateur entre :
- une position de repos avant actionnement,
- une position médiane, dans laquelle les moyens d'actionnement (43) libèrent le déplacement de la tige de poussée (37) depuis sa position initiale vers sa position intermédiaire, et
- une position terminale, dans laquelle les moyens d'actionnement (43) libèrent le déplacement de la tige de poussée (37) depuis sa position intermédiaire vers sa position finale.

4. Dispositif d'injection d'implant (1) selon la revendication précédente, dans lequel les moyens d'actionnement (43) comportent un élément d'indication (55) configuré pour indiquer à un utilisateur que le bouton d'actionnement (49) est dans sa position médiane, l'élément d'indication (55) comprenant de préférence un bouton secondaire (57) étant à affleurement du bouton d'actionnement (49) lorsque ce dernier est dans sa position médiane.

5. Dispositif d'injection d'implant (1) selon l'une quelconque des revendications 3 à 4, dans lequel les moyens d'actionnement (43) comportent un moyen de rappel (51) du bouton d'actionnement (49) vers sa position de repos, notamment une languette élastique (53) portée par le bouton d'actionnement (49), ou un ressort.

6. Dispositif d'injection d'implant (1) selon l'une quelconque des revendications 3 à 5, comportant des moyens anti-retour (59) empêchant le retour du bouton d'actionnement (49) respectivement depuis sa position médiane et depuis sa position terminale.

7. Dispositif d'injection d'implant (1) selon l'une quelconque des revendications précédentes, dans lequel les moyens d'actionnement (43) sont latéraux, par exemple mobiles par coulissement dans une direction radiale par rapport à l'axe longitudinal de la tige de poussée (37) ou par pivotement autour d'un axe orthogonal à l'axe longitudinal de la tige de poussée (37).

8. Dispositif d'injection d'implant (1) selon l'une quelconque des revendications précédentes, dans laquelle les moyens de butée intermédiaire (41) comportent un organe de butée axiale intermédiaire (47) permettant de maintenir la tige de poussée (37) dans la position intermédiaire, l'organe de butée axiale intermédiaire (47) étant configuré pour être entraîné par les moyens d'actionnement (43) pour libérer un déplacement de la tige de poussée (37) de la position intermédiaire vers la position finale.

9. Dispositif d'injection d'implant (1) selon la revendication 2, dans laquelle les moyens de butée initiale (73) comportent un organe de butée axiale initiale (75) permettant de maintenir la tige de poussée (37) dans la position initiale, l'organe de butée axiale initiale (75) étant configuré pour être entraîné par les moyens d'actionnement (43) pour libérer un déplacement de la tige de poussée (37) de la position initiale vers la position intermédiaire.

10. Dispositif d'injection d'implant (1) selon la revendication 8 ou 9, dans lequel les moyens d'actionnement (43) provoquent la rotation de l'un au moins parmi l'organe de butée axiale initiale (75) et l'organe de butée axiale intermédiaire (47) pour libérer respectivement un déplacement de la tige de poussée (37) de la position initiale vers la position intermédiaire et/ou de la position intermédiaire vers la position finale.

11. Dispositif d'injection d'implant (1) selon la revendication précédente, dans lequel l'un au moins parmi l'organe de butée axiale initiale (75) et l'organe de butée axiale intermédiaire (47) comporte un pion (80) disposé sur son extrémité distale, excentré par rapport à l'axe longitudinal de la tige de poussée (37), coopérant avec les moyens d'actionnement (43) pour provoquer la rotation de l'un au moins parmi l'organe de butée axiale initiale (75) et l'organe de butée axiale intermédiaire (47).

12. Dispositif d'injection d'implant (1) selon l'une quelconque des revendications précédentes, comprenant un boîtier de préhension (13, 19, 21, 23, 25) et un support (87) portant la tige de poussée (37) monté coulissant par rapport au boîtier de préhension (13, 19, 21, 23, 25), de préférence une douille coulissante (87) coopérant avec une rainure (89, 91) portée par le boîtier de préhension (13, 19, 21, 23, 25), le support (87) comprenant une surface d'appui (97) destinée à coopérer avec les moyens de butée intermédiaire (41) pour maintenir axialement la tige de poussée (37) dans la position intermédiaire.

13. Dispositif d'injection d'implant (1) selon l'une quelconque des revendications précédentes, dans lequel les moyens de poussée (39) comportent un ressort de poussée (45), prenant appui entre un boîtier de préhension (13, 19, 21, 23, 25) et la tige de poussée (37), de préférence disposé entre le boîtier de préhension (13, 19, 21, 23, 25) et le support (87) portant la tige de poussée (37).

14. Dispositif d'injection d'implant (1) selon l'une quelconque des revendications précédentes, comportant un élément de blocage amovible (103) des moyens de poussée (39), configuré pour maintenir le dispositif d'injection d'implant (1) dans une position de stockage, en particulier dans laquelle la tige de poussée (37) occupe sa position initiale.

15. Dispositif d'injection d'implant (1) selon l'une quelconque des revendications précédentes, comportant des moyens de verrouillage agencés pour bloquer la tige de poussée (37) dans sa position finale, position dans laquelle la tige de poussée (37) fait de préférence saillie vers la direction aval au-delà de l'extrémité de l'aiguille d'injection (3).

16. Dispositif d'injection d'implant (1) selon l'une quelconque des revendications précédentes, pouvant injecter une pluralité d'implants (9) et comprenant une pluralité de moyens de butée intermédiaire (41) respectifs permettant de maintenir la tige de poussée (37) dans une pluralité de positions intermédiaires respectives, lesquels s'opposent à la force exercée par les moyens de poussée (39) lorsque la course de la tige de poussée (37) atteint une distance prédéterminée respective correspondant à la longueur d'injection d'un implant (9) respectif.

## Patentansprüche

1. Implantat-Injektionsvorrichtung (1), **dadurch gekennzeichnet, dass** sie aufweist:
- eine Injektionsnadel (3),
- einen Aufnahmekörper (7) für mindestens ein erstes Implantat und ein zweites Implantat (9),
- Injektionsmittel (11), aufweisend:
- eine Schubstange (37), die stromaufwärts der in dem Aufnahmekörper (7) untergebrachten Implantate (9) angeordnet ist und konfiguriert ist, das erste Implantat und das zweite Implantat (9) durch die Injektionsnadel (3) zwischen einer Anfangsposition und einer Endposition, in der das erste Implantat und das zweite Implantat (9) injiziert werden, zu schieben,
- Schubmittel (39) an der Schubstange (37), die konfiguriert sind, eine Kraft auszuüben, die dazu neigt, die Schubstange (37) von der Anfangsposition in die Endposition zu bewegen,
- Zwischenanschlagmittel (41), die es ermöglichen, die Schubstange (37) in einer Zwischenposition zwischen der Anfangsposition und der Endposition zu halten, die der von den Schubmitteln (39) ausgeübten Kraft entgegenwirken, wenn der Hub der Schubstange (37) einen vorbestimmten Abstand erreicht, der der Injektionslänge des ersten Implantats (9) entspricht,
- Mittel (43) zur Betätigung durch einen Benutzer, die konfiguriert sind, eine Bewegung der Schubstange (37) von der Zwischenposition in Richtung der Endposition freizugeben.

2. Implantat-Injektionsvorrichtung (1) nach dem vorhergehenden Anspruch, mit Anfangsanschlagmitteln (73), die der von den Schubmitteln (39) ausgeübten Kraft entgegenwirken und es ermöglichen, die Schubstange (37) in der Ausgangsposition zu halten.

3. Implantat-Injektionsvorrichtung (1) nach einem der vorhergehenden Ansprüche, wobei die Betätigungsmittel (43) einen Betätigungsknopf (49) aufweisen, der durch Drücken durch einen Benutzer beweglich ist zwischen:
- einer Ruheposition vor der Betätigung,
- einer Mittelposition, in der die Betätigungsmittel (43) die Bewegung der Schubstange (37) von ihrer Ausgangsposition in ihre Zwischenposition freigeben, und
- einer Endposition, in der das Betätigungsmittel (43) die Bewegung der Schubstange (37) von ihrer Zwischenposition zu ihrer Endposition freigibt.

4. Implantat-Injektionsvorrichtung (1) nach dem vorhergehenden Anspruch, wobei die Betätigungsmittel (43) ein Anzeigeelement (55) aufweisen, das so konfiguriert ist, dass es einem Benutzer anzeigt, dass sich der Betätigungsknopf (49) in seiner Mittelposition befindet,
wobei das Anzeigeelement (55) vorzugsweise einen sekundären Knopf (57) aufweist, der bündig mit dem Betätigungsknopf (49) ist, wenn sich dieser in seiner Mittelposition befindet.

5. Implantat-Injektionsvorrichtung (1) nach einem der Ansprüche 3 bis 4, bei der die Betätigungsmittel (43) ein Mittel (51) zur Rückführung des Betätigungsknopfes (49) in seine Ruhestellung aufweisen, insbesondere eine elastische Zunge (53), die von dem Betätigungsknopf (49) getragen wird, oder eine Feder.

6. Implantat-Injektionsvorrichtung (1) nach einem der Ansprüche 3 bis 5, mit einer Rücklaufsperre (59), die verhindert, dass der Betätigungsknopf (49) aus seiner Mittelstellung bzw. aus seiner Endstellung zurückkehrt.

7. Implantat-Injektionsvorrichtung (1) nach einem der vorhergehenden Ansprüche, wobei die Betätigungsmittel (43) seitlich angeordnet sind und beispielsweise in einer radialen Richtung in Bezug auf die Längsachse der Schubstange (37) verschiebbar oder um eine Achse, die orthogonal zur Längsachse der Schubstange (37) verläuft, schwenkbar sind.

8. Implantat-Injektionsvorrichtung (1) nach einem der vorhergehenden Ansprüche, wobei die Zwischenanschlagmittel (41) ein Zwischen-Axialanschlagselement (47) aufweisen, das es ermöglicht, die Schubstange (37) in der Zwischenposition zu halten, wobei das Zwischen-Axialanschlagselement (47) konfiguriert ist, von den Betätigungsmitteln (43) angetrieben zu werden, um eine Bewegung der Schubstange (37) aus der Zwischenposition in die Endposition freizugeben.

9. Implantat-Injektionsvorrichtung (1) nach Anspruch 2, wobei die Anfangsanschlagmittel (73) ein Anfangs-Axialanschlagelement (75) aufweisen, das es ermöglicht, die Schubstange (37) in der Anfangsposition zu halten, wobei das Anfangs-Axialanschlagelement (75) konfiguriert ist, von den Betätigungsmitteln (43) angetrieben zu werden, um eine Bewegung der Schubstange (37) von der Anfangsposition in die Zwischenposition freizugeben.

10. Implantat-Injektionsvorrichtung (1) nach Anspruch 8 oder 9, wobei das Betätigungsmittel (43) die Drehung von mindestens einem von dem Anfangs-Axialanschlagselement (75) und dem mittleren Axialanschlagselement (47) bewirkt, um jeweils eine Bewegung der Schubstange (37) von der Anfangsposition in die mittlere Position und/oder von der mittleren Position in die Endposition freizugeben.

11. Implantat-Injektionsvorrichtung (1) nach dem vorhergehenden Anspruch, bei der mindestens eines von dem Anfangs-Axialanschlagselement (75) und dem mittleren axialen Axialanschlagselement (47) einen Stift (80) aufweist, der an seinem distalen Ende angeordnet ist, exzentrisch zur Längsachse der Schubstange (37) angeordnet ist und mit den Betätigungsmitteln (43) zusammenwirkt, um die Drehung von mindestens einem der Elemente des Anfangs-Axialanschlagelements (75) und des mittleren Axialanschlagselements (47) zu bewirken.

12. Implantat-Injektionsvorrichtung (1) nach einem der vorhergehenden Ansprüche, mit einem Greifgehäuse (13, 19, 21, 23, 25) und einem die Schubstange (37) tragenden Träger (87), der in Bezug auf das Greifgehäuse (13, 19, 21, 23, 25) verschiebbar angebracht ist, vorzugsweise eine Gleithülse (87), die mit einer vom Griffgehäuse (13, 19, 21, 23, 25) getragenen Nut (89, 91) zusammenwirkt, wobei der Träger (87) eine Auflagefläche (97) aufweist, die dazu bestimmt ist, mit den Zwischenanschlagmitteln (41) zusammenzuwirken, um die Schubstange (37) axial in der Zwischenposition zu halten.

13. Implantat-Injektionsvorrichtung (1) nach einem der vorhergehenden Ansprüche, bei der die Schubmittel (39) eine Schubfeder (45) aufweisen, die sich zwischen einem Greifgehäuse (13, 19, 21, 23, 25) und der Schubstange (37) abstützt, und die vorzugsweise zwischen dem Greifgehäuse (13, 19, 21, 23, 25) und dem die Schubstange (37) tragenden Träger (87) angeordnet ist.

14. Implantat-Injektionsvorrichtung (1) nach einem der vorhergehenden Ansprüche, mit einem lösbaren Blockierelement (103) des Schubmittels (39), das konfiguriert ist, die Implantat-Injektionsvorrichtung (1) in einer Lagerposition zu halten, insbesondere in der die Schubstange (37) ihre Anfangsposition einnimmt.

15. Implantat-Injektionsvorrichtung (1) nach einem der vorhergehenden Ansprüche, mit einer Verriegelungseinrichtung, die so angeordnet ist, dass sie die Schubstange (37) in ihrer Endposition blockiert, wobei die Schubstange (37) in dieser Position vorzugsweise in stromabwärtiger Richtung über das Ende der Injektionsnadel (3) hinausragt.

16. Implantat-Injektionsvorrichtung (1) nach einem der vorhergehenden Ansprüche, die eine Vielzahl von Implantaten (9) injizieren kann und eine Vielzahl von jeweiligen Zwischenanschlagmitteln (41) zum Halten der Schubstange (37) in einer Vielzahl von jeweiligen Zwischenpositionen aufweist, die der von den Schubmitteln (39) ausgeübten Kraft entgegenwirken, wenn der Hub der Schubstange (37) einen jeweiligen vorbestimmten Abstand erreicht, der der Injektionslänge eines jeweiligen Implantats (9) entspricht.

## Claims

1. Implant injection device (1), **characterised in that** it comprises:
- an injection needle (3),
- a receiver housing (7) for receiving at least a first implant and a second implant (9),
- injection means (11), comprising:
. a pushing rod (37), arranged upstream from said implants (9) housed in the receiver housing (7) and configured to push the first implant and the second implant (9) through the injection needle (3) between an initial position and a final position in which the first implant and the second implant (9) are injected,
. means (39) for pushing on the pushing rod (37), configured to exert a force tending to move the pushing rod (37) from the initial position to the final position,
. intermediate stop means (41) holding the pushing rod (37) in an intermediate position between the initial position and the final position, said means oppose the force exerted by the pushing means (39) when the stroke of the pushing rod (37) reaches a predetermined distance corresponding to the length of injection of the first implant (9),
. means (43) for actuation by a user, configured to release a displacement of the pushing rod (37) from the intermediate position to the final position.

2. Implant injection device (1) according to the preceding claim, comprising initial stop means (73) opposing the force exerted by the pushing means (39) and holding the pushing rod (37) in the initial position.

3. Implant injection device (1) according to any one of the preceding claims, wherein the actuation means (43) comprise an actuation button (49) which can be moved by pressing by a user between:
- a rest position before actuation,
- a middle position, in which the actuation means (43) release the displacement of the pushing rod (37) from its initial position to its intermediate position, and
- a terminal position, in which the actuation means (43) release the displacement of the pushing rod (37) from its intermediate position to its final position.

4. Implant injection device (1) according to the preceding claim, wherein the actuation means (43) comprise an indication element (55) configured to indicate to a user that the actuation button (49) is in its middle position, the indication element (55) preferably comprising a secondary button (57) flush with the actuation button (49) when the latter is in its middle position.

5. Implant injection device (1) according to claim 3 or 4, wherein the actuation means (43) comprise means (51) for returning the actuation button (49) to its rest position, in particular an elastic tab (53) carried by the actuation button (49), or a spring.

6. Implant injection device (1) according to any one of claims 3 to 5, comprising non-return means (59) preventing the actuation button (49) from returning respectively from its middle position and from its terminal position.

7. Implant injection device (1) according to any one of the preceding claims, wherein the actuation means (43) are lateral, for example movable by sliding in a radial direction relative to the longitudinal axis of the pushing rod (37) or by pivoting about an axis orthogonal to the longitudinal axis of the pushing rod (37).

8. Implant injection device (1) according to any one of the preceding claims, wherein the intermediate stop means (41) comprise an intermediate axial stop member (47) holding the pushing rod (37) in the intermediate position, the intermediate axial stop member (47) being configured to be driven by the actuation means (43) to release a displacement of the pushing rod (37) from the intermediate position to the final position.

9. Implant injection device (1) according to claim 2, wherein the initial stop means (73) comprise an initial axial stop member (75) holding the pushing rod (37) in the initial position, the initial axial stop member (75) being configured to be driven by the actuation means (43) to release a displacement of the pushing rod (37) from the initial position to the intermediate position.

10. Implant injection device (1) according to claim 8 or 9, wherein the actuation means (43) cause the rotation of at least one from the initial axial stop member (75) and the intermediate axial stop member (47) to release respectively a displacement of the pushing rod (37) from the initial position to the intermediate position and/or from the intermediate position to the final position.

11. Implant injection device (1) according to the preceding claim, wherein at least one from the initial axial stop member (75) and the intermediate axial stop member (47) comprises a pin (80) arranged on its distal end, offset relative to the longitudinal axis of the pushing rod (37), cooperating with the actuation means (43) to cause the rotation of at least one from the initial axial stop member (75) and the intermediate axial stop member (47).

12. Implant injection device (1) according to any one of the preceding claims, comprising a gripping unit (13, 19, 21, 23, 25) and a support (87) carrying the pushing rod (37) slidably mounted relative to the gripping unit (13, 19, 21, 23, 25), preferably a sliding bush (87) cooperating with a groove (89, 91) carried by the gripping unit (13, 19, 21, 23, 25), the support (87) comprising a bearing surface (97) intended to cooperate with the intermediate stop means (41) to hold the pushing rod (37) axially in the intermediate position.

13. Implant injection device (1) according to any one of the preceding claims, wherein the pushing means (39) comprise a thrust spring (45) resting between a gripping unit (13, 19, 21, 23, 25) and the pushing rod (37), preferably arranged between the gripping unit (13, 19, 21, 23, 25) and the support (87) carrying the pushing rod (37).

14. Implant injection device (1) according to any one of the preceding claims, comprising a removable locking element (103) to lock the pushing means (39), configured to hold the implant injection device (1) in a storage position, in particular in which the pushing rod (37) is in its initial position.

15. Implant injection device (1) according to any one of the preceding claims, comprising locking means arranged to block the pushing rod (37) in its final position, position in which the pushing rod (37) preferably projects towards the downstream direction past the end of the injection needle (3).

16. Implant injection device (1) according to any one of the preceding claims, which can inject a plurality of implants (9) and comprising a plurality of respective intermediate stop means (41) holding the pushing rod (37) in a plurality of respective intermediate positions, said means opposing the force exerted by the pushing means (39) when the stroke of the pushing rod (37) reaches a respective predetermined distance corresponding to the length of injection of a respective implant (9).
